# EUROPEAN PATENT APPLICATION

(11) **EP 4 772 207 A1**
(43) Date of publication of application: **08.07.2026**
(21) Application number: 24872240.7
(22) Date of filing: 25.09.2024
(51) Int. Cl.: A61M 1/18

(54) **ARTIFICIAL LUNG**

(30) Priority: 27.09.2023 JP 2023164701
(71) Applicant: Terumo Kabushiki Kaisha, Tokyo 151-0072 (JP)
(72) Inventor: SUZUKI, Ayato, Ashigarakami-gun, Kanagawa 259-0151 (JP); MORI, Takehisa, Ashigarakami-gun, Kanagawa 259-0151 (JP); SATO, Hitoshi, Ashigarakami-gun, Kanagawa 259-0151 (JP)
(74) Representative: KIPA AB
(86) International application number: PCT/JP2024/034078
(87) International publication number: WO 2025/070454

(57) **Abstract**

To suppress stagnation portion of blood flowing between a housing (10) and a hollow fiber membrane layer (30) and improve antithrombogenicity, an oxygenator (100) includes a housing (10) having a cylindrical shape and including a blood inflow port (14) and a blood outflow port (15), and a hollow fiber membrane layer (30) formed by winding a hollow fiber membrane and accommodated in a housing (10), the oxygenator in which blood flowing in from the blood inflow port (14) flows from one end toward the other end in a longitudinal direction of the housing (10) and flows out from the blood outflow port (15), in which the housing (10) includes a plurality of raised portions (16) (inner raised portions 16b) raised radially inward from an outer surface of an annular inner peripheral portion toward a surface of an innermost layer of the hollow fiber membrane layer (30) and extending from a distal end toward a proximal end of the housing (10), and a recess (17) adjacent to a downstream end of the raised portion (16) in a blood circulation direction, and formed continuously in a circumferential direction of the inner peripheral portion so as to face an outflow portion of the blood in a blood flow path (50) communicating with the blood outflow port (15).

## Description

### Technical Field

The present invention relates to an oxygenator.

### Background Art

Conventionally, an extracorporeal circulation system that assists circulation of blood and respiration of a patient has been widely used in order to temporarily maintain life at the time of open heart surgery of a heart disease or in a rapidly progressing circulation failure or cardiopulmonary arrest state.

The extracorporeal circulation system includes an oxygenator that is incorporated in an extracorporeal circulation circuit including a blood removal path, a blood supply path and the like and exchanges gas with blood, and a pump that is incorporated in the extracorporeal circulation circuit and sends blood to the oxygenator.

In the extracorporeal circulation system, the oxygenator performs gas exchange (gives oxygen to blood and removes carbon dioxide) on the removed blood by a hollow fiber membrane layer disposed in the oxygenator, and sends the blood to the blood supply path (for example, refer to Patent Literature 1).

### Citation List

### Patent Literature

Patent Literature 1: German Patent Application Publication No. 10341221

### Summary of Invention

### Technical Problem

In a case where an extracorporeal circulation system is used for a long period of time, there is a problem that thrombus occurs in the oxygenator. In the oxygenator, a hollow fiber membrane layer is accommodated in a cylindrical housing, and a blood flow stagnates due to winding disturbance or the like of hollow fiber membranes in the hollow fiber membrane layer, so that a thrombus may be formed between the hollow fiber membrane layer and the housing and the like.

The present invention has been made in view of the above problems, and an object thereof is to provide an oxygenator that suppresses a stagnation portion of blood flowing between a housing and a hollow fiber membrane layer and improves antithrombogenicity.

### Solution to Problem

The above object of the present invention is achieved by the following means (1) to (6).
(1) An oxygenator including a housing having a cylindrical shape and including a blood inflow port and a blood outflow port, and a hollow fiber membrane layer formed by winding a hollow fiber membrane and accommodated in the housing, the oxygenator in which blood flowing in from the blood inflow port flows from one end toward the other end in a longitudinal direction of the housing and flows out from the blood outflow port, in which the housing includes a plurality of raised portions that is raised radially inward from an outer surface of an annular inner peripheral portion toward a surface of an innermost layer of the hollow fiber membrane layer and extends from a distal end toward a proximal end of the housing, and a recess adjacent to a downstream end of the raised portions in a blood circulation direction and continuously formed in a circumferential direction of the inner peripheral portion so as to face an outflow portion of the blood in a blood flow path communicating with the blood outflow port.
(2) The oxygenator according to (1) described above, in which a raised portion has a chevron shape having a smooth outer peripheral surface in which an amount raised outward from a center decreases in a transverse sectional view orthogonal to the longitudinal direction.
(3) The oxygenator according to (1) or (2) described above, in which the raised portion is smoothly connected with the raised portion adjacent in the circumferential direction.
(4) The oxygenator according to any one of (1) to (3) described above, in which the hollow fiber membrane layer includes a gas exchange unit that performs gas exchange of the blood and a heat exchange unit that performs heat exchange of the blood, and the raised portions are disposed at least to face the surface of the innermost layer of the hollow fiber membrane layer forming the gas exchange unit.
(5) The oxygenator according to any one of (1) to (4) described above, in which the recess includes a straight portion an outer diameter of which gradually decreases from the downstream end of the raised portions in a blood circulation direction, and a curved portion disposed adjacent to the straight portion and having an arc shape directed radially outward with a predetermined curvature radius.
(6) The oxygenator according to any one of (1) to (5) described above, in which the raised portions are formed continuously or discontinuously from the distal end to the proximal end of the housing.

### Advantageous Effects of Invention

According to an oxygenator of the present embodiment, in a device configuration in which blood flows from one end toward the other end in a longitudinal direction of a housing from a blood inflow port to a blood outflow port, a raised portion is provided in which a circulation direction of the blood flowing through an innermost layer of a hollow fiber membrane layer is formed from one end toward the other end in the longitudinal direction of the housing. The raised portion increases a flow velocity while defining the flow of the blood flowing in the innermost layer of the hollow fiber membrane layer in the housing to flow from one end toward the other end in the longitudinal direction of the housing. Therefore, by increasing the flow velocity of the blood flowing through the innermost layer of the hollow fiber membrane layer by the raised portion, the oxygenator can suppress the formation of the stagnation portion of the blood in the innermost layer of the hollow fiber membrane layer to enhance antithrombogenicity. In the blood flowing in the housing in the longitudinal direction, the flow of the blood at a position of an outflow portion in a blood flow path at which the circulation direction of the blood can be rapidly changed by a recess becomes smooth, and it is possible to suppress the formation of the stagnation portion at a place at which the blood easily stagnates.

### Brief Description of Drawings

Fig. 1 is a longitudinal sectional view of an oxygenator of a first embodiment according to the present embodiment.
Fig. 2 is a schematic transverse sectional view of the oxygenator according to the present embodiment.
Fig. 3 is a partially enlarged view of the periphery of a raised portion of the oxygenator according to the present embodiment.
Fig. 4A is a diagram illustrating a mode of an outer raised portion of the oxygenator according to the present embodiment.
Fig. 4B is a diagram illustrating Modification 1 of the outer raised portion of the oxygenator according to the present embodiment.
Fig. 4C is a diagram illustrating Modification 2 of the outer raised portion of the oxygenator according to the present embodiment.
Fig. 5A is a diagram illustrating a mode of an inner raised portion of the oxygenator according to the present embodiment.
Fig. 5B is a diagram illustrating Modification 1 of the inner raised portion of the oxygenator according to the present embodiment.
Fig. 5C is a diagram illustrating Modification 2 of the inner raised portion of the oxygenator according to the present embodiment.
Fig. 6 is a diagram illustrating the periphery of a recess of the oxygenator according to the present embodiment.
Fig. 7 is a longitudinal sectional view of an oxygenator of a second embodiment according to the present embodiment.

### Description of Embodiments

Hereinafter, a mode for carrying out the present invention will be described in detail with reference to the drawings. Embodiments herein described are illustrated to embody the technical idea of the present invention and do not limit the present invention. Other embodiments, examples, technical operations and the like that could be conceived by those skilled in the art without departing from the gist of the present invention are all included in the scope and gist of the present invention and included in the invention recited in claims and the scope of equivalents thereof.

Furthermore, for the purpose of illustration and for ease of comprehension, the scale, aspect ratio, shape and the like in the drawings attached to the present specification may be changed from actual ones as appropriate and illustrated schematically; however, the drawings are examples and do not limit the interpretation of the present invention.

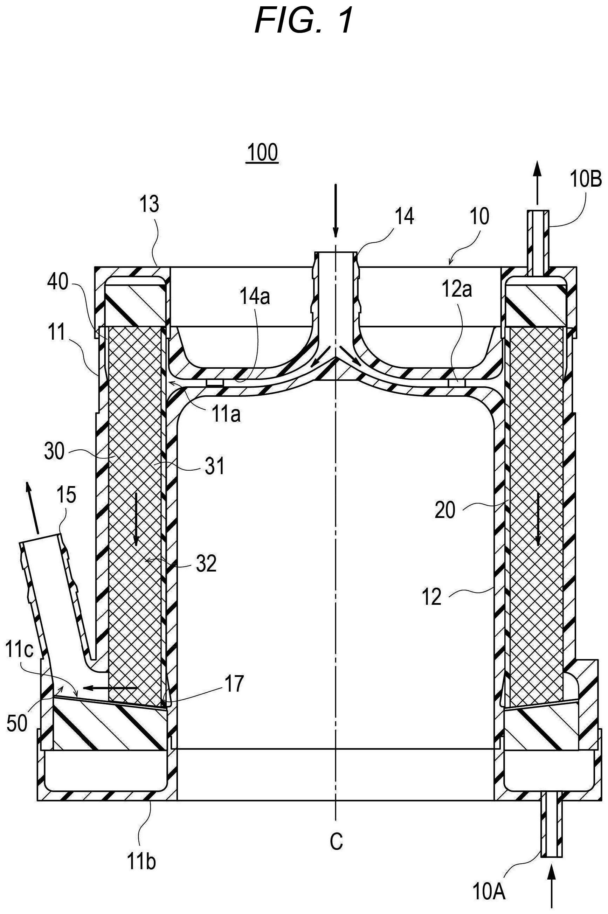

In the present specification, the following directions are defined for convenience of description. In Fig. 1, a "longitudinal direction" is a direction along a central axis C of an oxygenator 100. A "radial direction" is a direction away from or approaching the central axis C in an axial orthogonal cross-section (transverse section) with the central axis C of the oxygenator 100 as a reference axis. A "circumferential direction" is a rotational direction with the central axis C of the oxygenator 100 as a reference axis. The central axis C substantially coincides with central axes of an outer cylinder 11 and an inner cylinder 12 forming a housing 10.

Note that, in the following description, ordinal numbers such as "first" and "second" are used for convenience and do not define any order unless otherwise specified.

The inventor of the present application has started development of a novel oxygenator in order to solve the problem of thrombus formation due to blood stagnation, which is a problem of the conventional device. The inventor of the present application has fundamentally reviewed a blood flow path and a circulation direction of blood in the oxygenator, and has found that partial blood stagnation can be suppressed by allowing the blood that flows in from a blood inflow port to circulate from one end toward the other end in a longitudinal direction of a housing and flow out from a blood outflow port.

In the oxygenator including a new blood flow path, blood stagnation in an outermost layer is eliminated and antithrombogenicity is obtained, but on the other hand, a new problem has been found that blood stagnation may newly occur in a portion where the housing and a hollow fiber membrane layer are in contact with each other.

The inventor of the present application has further studied this new problem and found that the above problem can be solved by adopting a structure in which blood flowing through an outermost layer and an innermost layer of the hollow fiber membrane layer has a flow from one end toward the other end in the longitudinal direction of the housing. The present invention is a novel oxygenator developed based on the above findings. Note that, the outermost layer and the innermost layer of the hollow fiber membrane layer include not only an outermost layer surface and an innermost layer surface of the hollow fiber membrane layer itself but also an innermost layer surface of a core serving as a winding axis when the hollow fiber membrane layer is formed. Therefore, the blood flowing through the outermost layer or the innermost layer of the hollow fiber membrane layer refers to the blood flowing between the hollow fiber membrane layer (or the core) and components of the housing.

### [First Embodiment]

An oxygenator 100 according to a first embodiment of the present invention will be described.

The oxygenator 100 is incorporated in an extracorporeal circulation circuit and exchanges gas with blood. The oxygenator 100 includes a membrane oxygenator that exchanges gas with the blood by a hollow fiber membrane 31.

As illustrated in Fig. 1, the oxygenator 100 includes a cylindrical housing 10, a core 20 accommodated in an accommodation space 11a of the housing 10, a hollow fiber membrane layer 30 formed by a bundle of the hollow fiber membranes 31 wound around the core 20 to be stacked, a blood chamber 40 formed between the housing 10 and the hollow fiber membrane layer 30, and a blood flow path 50 serving as a flow path of the blood in the housing 10. Note that, an outer peripheral surface of the core 20 and an inner peripheral surface of the hollow fiber membrane layer 30 are partially in contact with each other. In other words, the blood chamber 40 is formed by a void in a portion in which the outer peripheral surface of the core 20 and the inner peripheral surface of the hollow fiber membrane layer 30 are not in contact with each other.

### <Housing>

The housing 10 is a casing that forms a main body of the oxygenator 100. As illustrated in Fig. 1, the housing 10 includes a cylindrical outer cylinder 11 extending in a longitudinal direction (vertical direction in Fig. 1), an inner cylinder 12 accommodated in the outer cylinder 11, a lid 13 airtightly coupled to outer peripheral ends of the outer cylinder 11 and the inner cylinder 12, a blood inflow port 14 for allowing the blood to flow into the housing 10, a blood outflow port 15 for allowing the blood circulating in the housing 10 to flow out, a raised portion 16 raised radially inward or outward on an inner peripheral surface of the outer cylinder 11, an outer peripheral surface of the inner cylinder 12 or the like, and a recess 17 disposed at a downstream end of the raised portion 16 formed in the inner cylinder 12 in a blood circulation direction.

As illustrated in Fig. 1, the outer cylinder 11 has a cylindrical shape, and includes the accommodation space 11a for accommodating the core 20 and the hollow fiber membrane layer 30 therein. The blood outflow port 15 is provided at a proximal end of the outer cylinder 11 so as to extend in a direction intersecting the longitudinal direction of the outer cylinder 11.

As illustrated in Fig. 1, the inner cylinder 12 has a cylindrical shape having a diameter smaller than that of the outer cylinder 11. The inner cylinder 12 functions as an annular inner peripheral portion of the housing 10. On a distal end side of the inner cylinder 12, a plurality of protrusions 12a for positioning when arranging the blood inflow port 14 and providing a gap serving as a guide portion 14a is arranged at predetermined intervals in a circumferential direction.

The lid 13 is a bottomed annular member having a concave cross-section, and airtightly closes a distal end in the longitudinal direction of the outer cylinder 11 in a state in which the inner cylinder 12, the blood inflow port 14, the core 20, and the hollow fiber membrane layer 30 are accommodated in the outer cylinder 11.

An outer bottom 11b of the oxygenator 100 is provided with a gas inflow port 10A that allows gas for the gas exchange of the blood to flow into the housing 10. The lid 13 is provided with a gas outflow port 10B that allows gas after the gas exchange to flow out. From the viewpoint of improving efficiency of the gas exchange in the blood, the gas inflow port 10A is preferably disposed at a position (proximal end side of the outer cylinder 11 on a downstream side in the circulation direction of the blood) at which the gas can flow into the blood chamber 40 opposed to the circulation direction of the blood circulating in the accommodation space 11a (blood chamber 40). The gas outflow port 10B is preferably disposed at a position substantially opposite to the gas inflow port 10A in the longitudinal direction (on the distal end side of the outer cylinder 11 on an upstream side in the circulation direction of the blood). Note that, the arranging positions of the gas inflow port 10A and the gas outflow port 10B are not limited to the positions illustrated in Fig. 1 as long as they are positions at which gas exchange of blood is possible in the accommodation space.

The blood inflow port 14 is attachable to and detachable from the housing 10, and is disposed on the distal end side of the inner cylinder 12 of the housing 10. The blood inflow port 14 is connected to a blood removal path in the extracorporeal circulation circuit of the extracorporeal circulation system, and allows blood to flow into the housing 10. A distal end opening serving as an inflow port of the blood inflow port 14 is formed to face the distal end side in the longitudinal direction of the housing 10. The blood flowing in from the blood inflow port 14 flows into the blood chamber 40 through the guide portion 14a formed between the same and the distal end side of the inner cylinder 12.

The blood outflow port 15 extends in a direction intersecting the circulation direction of the blood flowing in the housing 10 (longitudinal direction of the housing 10) and is disposed on an outer peripheral portion of the outer cylinder 11. The blood outflow port 15 can be provided at a proximal end of the housing 10. The blood outflow port 15 is connected to a blood supply path in the extracorporeal circulation circuit of the extracorporeal circulation system, and causes the blood circulating in the housing 10 to flow out to the blood supply path. In the mode illustrated in Fig. 1, a distal end opening of the blood outflow port 15 is formed to face the distal end side of the housing 10, similarly to the distal end opening serving as the inflow port of the blood inflow port 14. However, the direction of the distal opening of the blood outflow port 15 is not limited thereto.

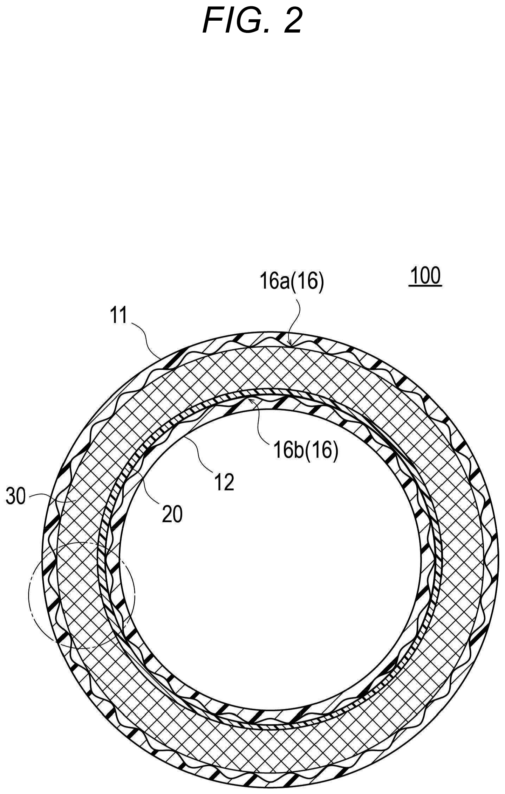

The raised portion 16 is formed to extend from one end toward the other end in the longitudinal direction of the housing 10. As illustrated in Fig. 2, the raised portions 16 include an outer raised portion 16a that is raised radially inward from the inner peripheral surface of the outer cylinder 11 toward a surface of an outermost layer of the hollow fiber membrane layer 30 and extends from the distal end toward the proximal end of the housing 10, and an inner raised portion 16b that is raised radially outward from the inner peripheral surface of the inner cylinder 12 toward a surface of an innermost layer of the hollow fiber membrane layer 30 and extends from the distal end toward the proximal end of the housing 10. A plurality of raised portions 16 is formed with respect to the housing 10. The raised portion 16 guides the circulation direction of the blood flowing through the blood chamber 40 in the longitudinal direction (for example, a direction from the distal end toward the proximal end) of the housing 10.

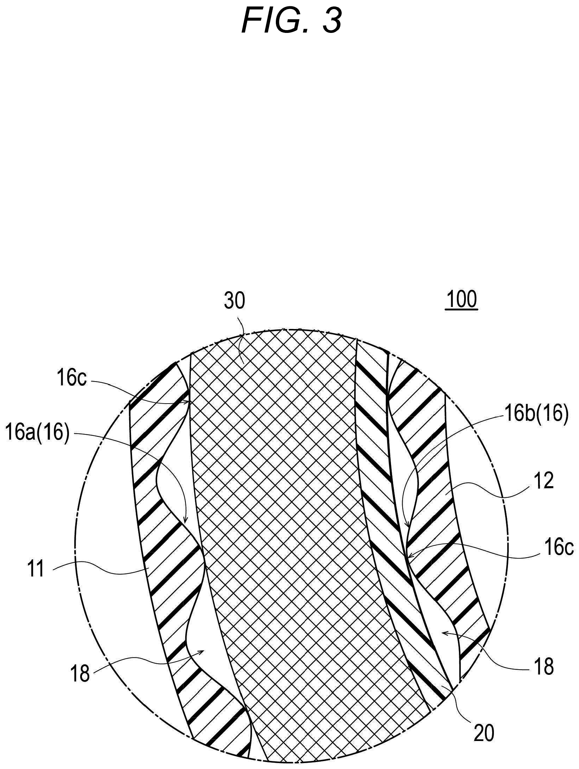

As illustrated in Fig. 3, the raised portion 16 can be formed such that an apex 16c abuts an outer peripheral surface (surface of the outermost layer) of the hollow fiber membrane layer 30 or an inner peripheral surface of the core 20 around which the hollow fiber membrane 31 is wound. As a result, in the oxygenator 100, a circulation path 18 of the blood is formed between the raised portions 16 adjacent to each other in the circumferential direction of the housing 10. The circulation path 18 extends in the longitudinal direction of the housing 10 and defines the circulation direction of the blood so that the blood in the housing 10 flows from the distal end toward the proximal end of the housing 10. The circulation path 18 functions as a stagnation suppression portion that forms a flow of the blood in the longitudinal direction from the distal end toward the proximal end of the housing 10 and suppresses formation of a stagnation portion of the blood in the housing 10.

In this manner, since the oxygenator 100 can form the flow of the blood in the outermost layer of the hollow fiber membrane layer 30 in the blood chamber 40 of the housing 10, the flow of the blood in the longitudinal direction from the distal end side toward the proximal end side of the housing 10, formation of the stagnation portion in the blood chamber 40 is suppressed, and antithrombogenicity can be improved.

In consideration of formation of the circulation path 18, position fixing of the hollow fiber membrane layer 30 and the like, the apex 16c of the raised portion 16 can abut a part of the hollow fiber membrane layer 30 or the core 20 to form the circulation path 18. Note that, the hollow fiber membrane layer 30 may be wound around the core 20 as illustrated in Fig. 1, but it is preferable to form by directly winding the hollow fiber membrane 31 around the inner cylinder 12. In this case, the core 20 is formed as a part of the inner cylinder 12. It is sufficient that the raised portion 16 can form the flow of the blood from the distal end toward the proximal end of the housing 10 in an outermost layer or an innermost layer of the hollow fiber membrane layer 30 in the blood chamber 40. Therefore, the raised portion 16 may be disposed with a slight gap between the apex 16c and the hollow fiber membrane layer 30 or the core 20 to such an extent that the stagnation portion is not formed without hindering the flow of the blood.

Next, modes of the outer raised portion 16a and the inner raised portion 16b forming the raised portion 16 will be described.

As illustrated in Fig. 3, the outer raised portion 16a and the inner raised portion 16b have a chevron shape having a smooth outer peripheral surface in which an amount raised outward from the center decreases in a transverse sectional view orthogonal to the longitudinal direction. As illustrated in Fig. 3, the outer raised portion 16a and the inner raised portion 16b are smoothly connected to the adjacent raised portions 16 in the circumferential direction. As illustrated in Fig. 3, the outer raised portions 16a and the inner raised portions 16b can be formed in a wave shape in which an uneven shape is continuous in the circumferential direction of the inner peripheral surface of the housing 10. As a result, the blood is less likely to stagnate in the blood chamber 40 formed in the accommodation space 11a, and the antithrombogenicity can be effectively enhanced. Note that, it is sufficient that a plurality of raised portions 16 is formed at least to be raised radially inward from the inner peripheral surface of the housing 10 and to extend from the distal end toward the proximal end of the housing 10, so that the shape thereof is not limited to the wave shape as illustrated in Figs. 2 and 3.

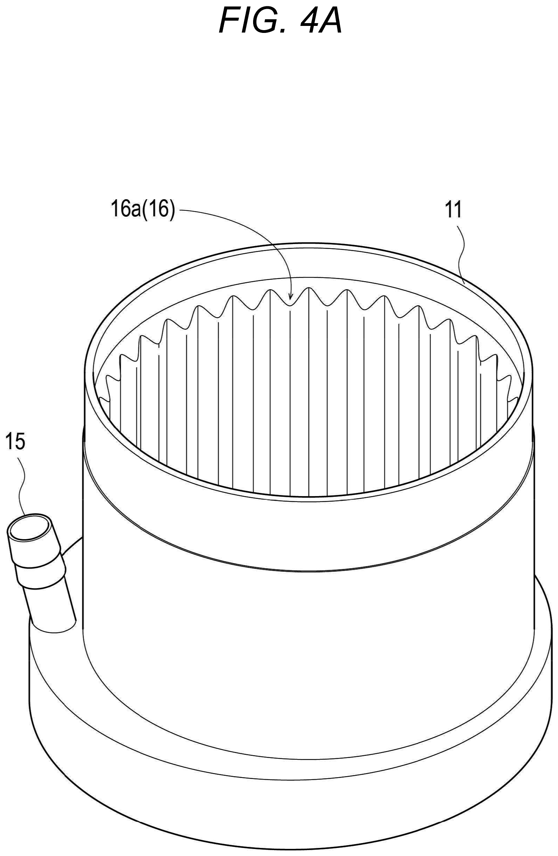

As illustrated in Figs. 4A to 4C, it is sufficient that a plurality of outer raised portions 16a is formed at least to be raised radially inward from the inner peripheral surface of the outer cylinder 11 and to extend from the distal end toward the proximal end of the outer cylinder 11.

As illustrated in Fig. 4A, a plurality of outer raised portions 16a can be formed so as to extend continuously from the distal end toward the proximal end of the outer cylinder 11 in the longitudinal direction and at predetermined intervals in the circumferential direction of the outer cylinder 11.

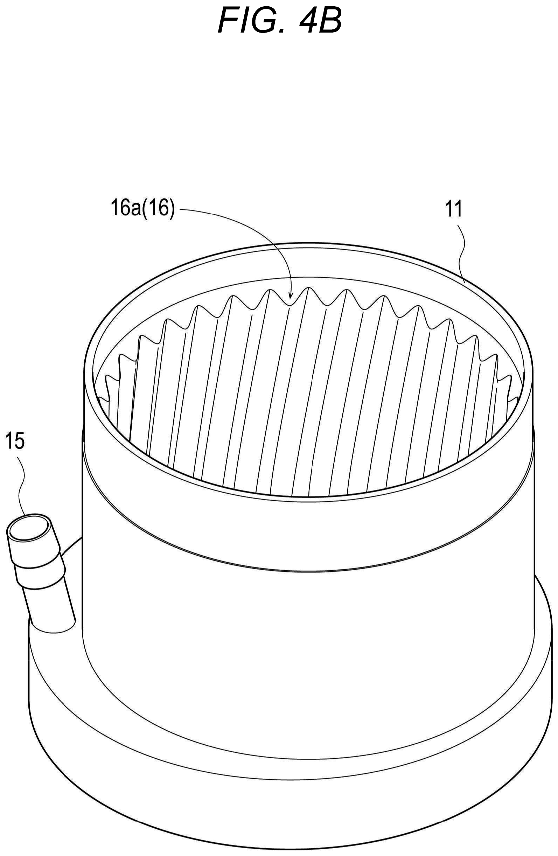

As illustrated in Fig. 4B, as Modification 1, a plurality of outer raised portions 16a can be formed so as to extend continuously from the distal end toward the proximal end of the outer cylinder 11 so as to be inclined with respect to the longitudinal direction and at predetermined intervals in the circumferential direction of the outer cylinder 11.

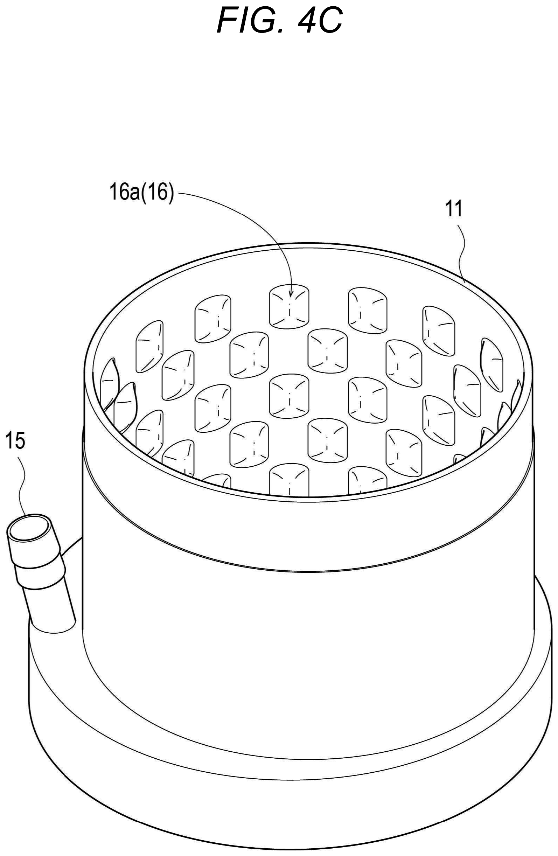

As illustrated in Fig. 4C, as Modification 2, the outer raised portions 16a can be formed so as not to be continuous from the distal end toward the proximal end of the outer cylinder 11 so as to be discontinuous at predetermined intervals in the longitudinal direction. As illustrated in Fig. 4C, in a case where the outer raised portions 16a are discontinuously formed, if they are formed in a staggered arrangement in the circumferential direction of the outer cylinder 11, the function of the circulation path 18 is easily exhibited, which is preferable. Note that, in Fig. 4C, the outer raised portions 16a illustrated in Fig. 4A are discontinuously formed from the distal end toward the proximal end of the outer cylinder 11, but this can also be applied to the mode illustrated in Fig. 4B.

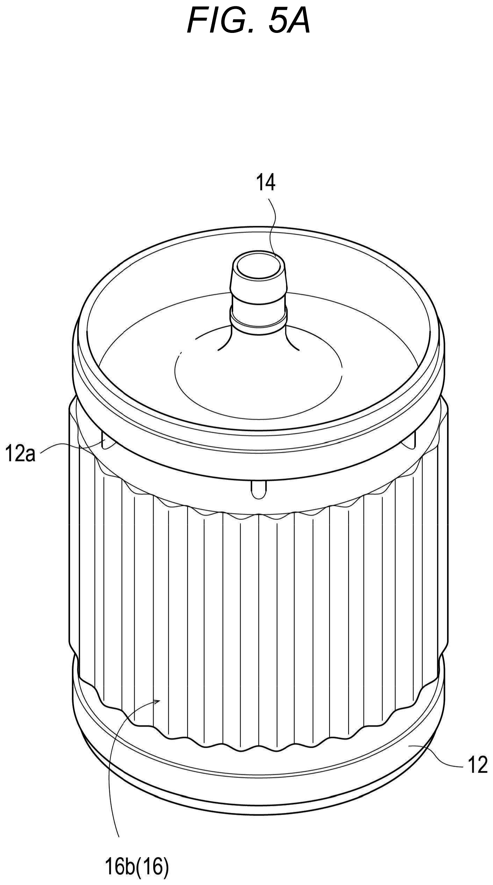

As illustrated in Figs. 5A to 5C, it is sufficient that a plurality of inner raised portions 16b is formed at least to be raised radially outward from the outer peripheral surface of the inner cylinder 12 and to extend from the distal end toward the proximal end of the inner cylinder 12.

As illustrated in Fig. 5A, a plurality of inner raised portions 16b can be formed so as to extend continuously from the distal end toward the proximal end of the inner cylinder 12 in the longitudinal direction and at predetermined intervals in the circumferential direction of the inner cylinder 12.

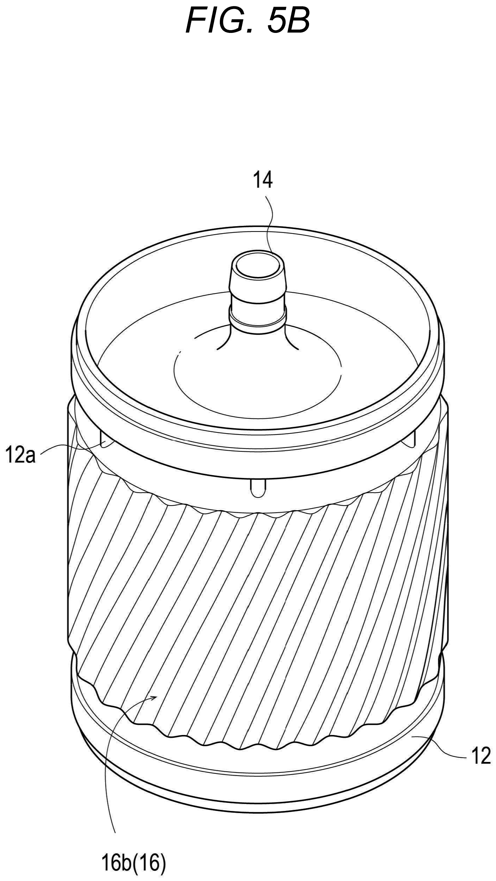

As illustrated in Fig. 5B, as Modification 1, a plurality of inner raised portions 16b can be formed so as to extend continuously from the distal end toward the proximal end of the inner cylinder 12 so as to be inclined with respect to the longitudinal direction and at predetermined intervals in the circumferential direction of the inner cylinder 12.

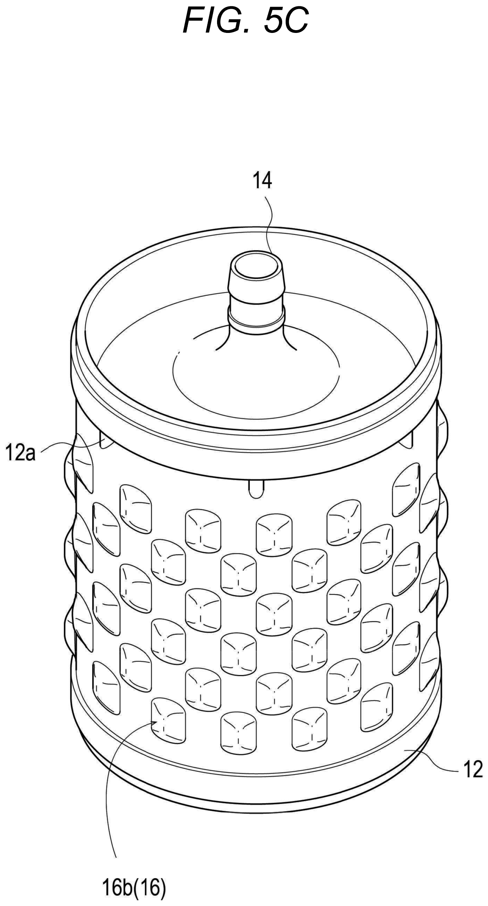

As illustrated in Fig. 5C, as Modification 2, the inner raised portions 16b can be formed so as not to be continuous from the distal end toward the proximal end of the inner cylinder 12 so as to be discontinuous at predetermined intervals in the longitudinal direction. As illustrated in Fig. 5C, in a case where the inner raised portions 16b are discontinuously formed, if they are formed in a staggered arrangement in the circumferential direction of the outer cylinder 11, the function of the circulation path 18 is easily exhibited, which is preferable. Note that, in Fig. 4C, the outer raised portions 16a illustrated in Fig. 4A are discontinuously formed from the distal end toward the proximal end of the outer cylinder 11, but this can also be applied to the mode illustrated in Fig. 4B.

In any of the modes illustrated in Figs. 4A to 5C, the raised portion 16 can form the circulation path 18 that allows the blood flowing in the blood chamber 40 to have a flow from one end toward the other end in the longitudinal direction of the housing 10, so that an effect of suppressing the formation of the stagnation portion and an effect of enhancing the antithrombogenicity can be obtained. From the viewpoint of increasing a flow velocity of the blood flowing around the hollow fiber membrane layer 30, it is preferable that both the outer raised portion 16a and inner raised portion 16b are provided as the raised portions 16, but it is also possible to provide either one. That is, in a case where only the outer raised portion 16a is provided, it is possible to suppress occurrence of blood stagnation at a place at which the hollow fiber membrane layer 30 and the outer cylinder 11 are in contact with each other, and in a case where only the inner raised portion 16b is provided, it is possible to suppress occurrence of stagnation at a place at which the hollow fiber membrane layer 30 and the inner cylinder 12 are in contact with each other and in the vicinity of the flow path to the blood outflow port 15.

The raised portion 16 is integrally formed with the inner peripheral surface of the outer cylinder 11 and the outer peripheral surface of the inner cylinder 12. However, the raised portion 16 may be formed separately from the outer cylinder 11 and the inner cylinder 12, and may be fixed to the inner peripheral surface of the outer cylinder 11 and the outer peripheral surface of the inner cylinder 12 with an adhesive or the like.

In a case of the modes illustrated in Figs. 4C and 5C, it is preferable that the outer raised portion 16a and the inner raised portion 16b have an R shape at a portion facing the longitudinal direction so as not to hinder the flow of the blood in the longitudinal direction. This R shape may be either convex or concave toward the hollow fiber membrane layer 30 as long as this does not hinder the flow of the blood in the longitudinal direction and allows the blood to smoothly flow. As a result, since a surface hindering the flow of the blood in the axial direction can be formed more smoothly in the R shape, the occurrence of thrombus can be suppressed. When the hollow fiber membrane layer 30 is inserted or replaced, a contact surface with the layer is smooth, so that deterioration in insertability, damage to a fiber and the like can be prevented.

The recess 17 is adjacent to a downstream end in the blood circulation direction of the inner raised portion 16b, which is the inner peripheral portion of the housing 10, and is formed continuously in the circumferential direction of the inner peripheral portion so as to face an outflow portion of the blood in the blood flow path 50.

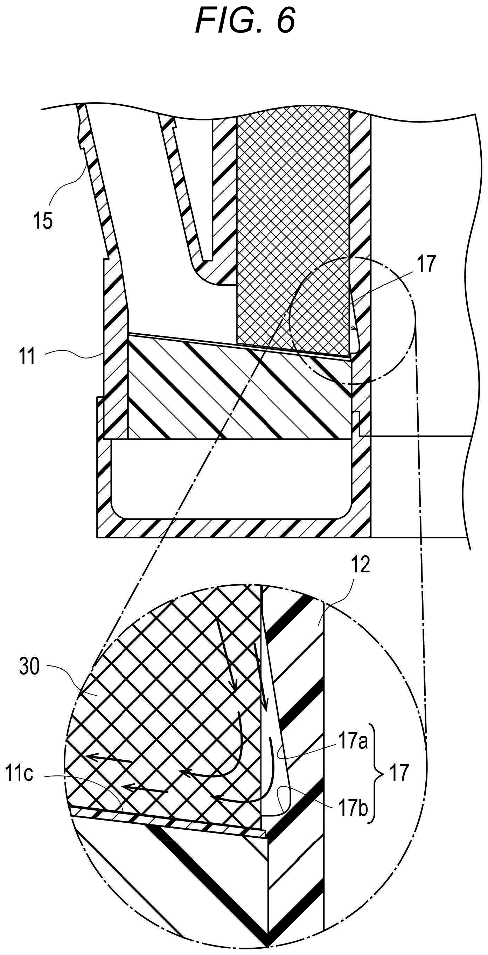

As illustrated in Fig. 6, the recess 17 is formed of a straight portion 17a of which outer diameter gradually decreases from the downstream end in the blood circulation direction of the raised portion 16, and a curved portion 17b disposed adjacent to the straight portion 17a and having an arc shape directed radially outward with a predetermined curvature radius. As illustrated in Fig. 6, one end of the straight portion 17a is disposed adjacent to the downstream end of the inner raised portion 16b in the blood circulation direction, an outer diameter thereof gradually decreases radially inward toward a proximal end, and the other end is connected to the curved portion 17b. The curved portion 17b includes one end connected to the proximal end of the straight portion 17a, is curved so as to draw an arc shape directed radially outward with a predetermined curvature radius, and includes the other end connected to an inner bottom 11c of the outer cylinder 11.

By providing the recess 17 at a position facing the outflow portion (for example, a place at which a flow in the longitudinal direction of the housing 10 changes to a direction intersecting the longitudinal direction) in the blood flow path 50 at which the circulation direction of the blood can rapidly change, it is possible to guide the blood in the circulation direction while suppressing stagnation of the blood. As illustrated in Fig. 1, the recess 17 can be disposed at a position facing an inflow port (proximal end opening) of the blood outflow port 15 serving as the outflow portion at which the circulation direction of the blood rapidly changes from the longitudinal direction to the direction intersecting the longitudinal direction. As a result, the blood flows in the longitudinal direction of the housing 10 and then is guided to the proximal end opening of the blood outflow port 15 by the recess 17. Therefore, in the oxygenator 100, the flow of the blood at a position of the outflow portion in the blood flow path 50 at which the circulation direction of the blood can rapidly change becomes smooth, and formation of the stagnation portion at the place at which the blood easily stagnates can be suppressed.

The housing 10 is preferably transparent to the extent that the blood flow therein is visible. Note that, the term "transparent" in the present specification includes colorless transparent, colored transparent, and translucent.

A material forming the housing 10 is not particularly limited, and for example, polyolefins such as polyethylene and polypropylene, ester-based resins such as polyethylene terephthalate, styrene-based resins such as polystyrene, MS resin, and MBS resin, polycarbonate and the like can be used.

### <Core>

The core 20 has a cylindrical shape, and is accommodated in the housing 10 in a state in which the hollow fiber membrane 31 is wound and the hollow fiber membrane layer 30 is included. The core 20 is attached to cover the inner cylinder 12. Note that, the core 20 may be provided integrally with the inner cylinder 12. In this case, the hollow fiber membrane 31 is directly wound around the inner cylinder 12, and the inner cylinder 12 functions as the core 20.

The material forming the core 20 is not particularly limited, and for example, similarly to the inner cylinder 12, polyolefins such as polyethylene and polypropylene, ester-based resins such as polyethylene terephthalate, styrene-based resins such as polystyrene, MS resin, and MBS resin, polycarbonate and the like can be used.

### <Hollow Fiber Membrane Layer>

As illustrated in Fig. 1, the hollow fiber membrane layer 30 is formed in a state in which the hollow fiber membranes 31 are wound around the core 20 and stacked in multiple layers.

As illustrated in Fig. 1, the hollow fiber membrane layer 30 includes a gas exchange unit 32.

The gas exchange unit 32 is formed of a bundle of the hollow fiber membranes 31. In the gas exchange unit 32, oxygen that circulates in the gas exchange unit 32 is diffused to a blood side when passing through the hollow fiber membrane 31. Carbon dioxide in the blood that circulates in the gas exchange unit 32 is discharged into a lumen of the hollow fiber membrane 31. As a result, in the gas exchange unit 32, gas exchange of oxygen and carbon dioxide is performed with the blood via the hollow fiber membrane 31.

Oxygen that flows in from the gas inflow port 10A is subjected to the gas exchange with carbon dioxide in the blood in the gas exchange unit 32, and carbon dioxide subjected to the gas exchange is discharged out of the oxygenator 100 through the gas outflow port 10B.

The hollow fiber membrane layer 30 has a stacked structure obtained by winding the hollow fiber membranes 31 around the core 20 in multiple layers, and thus can be formed by, for example, winding one or a plurality of bundled hollow fiber membranes 31 with respect to the core 20 in a spiral shape so as to be inclined with respect to the central axis C of the core 20 and substantially parallel to the axis of the core 20. The hollow fiber membrane 31 is formed by forming a large number of hollow fibers having a gas exchange function into a tubular shape.

A forming material of the hollow fiber membrane 31 is not particularly limited as long as the gas exchange with the blood can be performed, and for example, a hydrophobic polymer material such as polypropylene, polyethylene, polysulfone, polyacrylonitrile, polytetrafluoroethylene, or polymethylpentene can be used.

Next, the flow of the blood in the oxygenator 100 will be described.

In the oxygenator 100, the blood circulates in the blood flow path 50 from the blood inflow port 14 to the blood outflow port 15 through the guide portion 14a and the blood chamber 40 in this order.

The blood that flows in from the blood inflow port 14 flows through the guide portion 14a and is guided to the blood chamber 40.

The blood guided to the blood chamber 40 circulates between the inner peripheral surface of the outer cylinder 11 and the outermost layer of the hollow fiber membrane layer 30, between the outer peripheral surface of the inner cylinder 12 and the inner peripheral surface of the core 20 in the innermost layer of the hollow fiber membrane layer 30, and in the hollow fiber membrane layer 30 to expand to flow from the distal end side toward the proximal end side of the housing 10. When passing through the hollow fiber membrane layer 30, the blood exchanges gas with oxygen in the gas exchange unit 32 while moving through the gap of the hollow fiber membranes 31.

The blood flowing between the inner peripheral surface of the outer cylinder 11 and the outermost layer of the hollow fiber membrane layer 30 passes through the circulation path 18 formed of the outer raised portion 16a to flow from the distal end toward the proximal end of the housing 10 in the outermost layer of the hollow fiber membrane layer 30. The blood flowing between the outer peripheral surface of the inner cylinder 12 and the inner peripheral surface of the core 20 of the innermost layer of the hollow fiber membrane layer 30 passes through the circulation path 18 formed of the inner raised portion 16b to flow from the distal end toward the proximal end of the housing 10 in the innermost layer of the hollow fiber membrane layer 30. Therefore, in the oxygenator 100, the formation of the stagnation portion of the blood in the outermost layer and the innermost layer of the hollow fiber membrane layer 30 is suppressed, and the thrombus is less likely to be formed.

The blood subjected to the gas exchange flows out of the oxygenator 100 from the blood outflow port 15 communicating with the blood chamber 40 and returns to the human body via the extracorporeal circulation circuit.

Next, a manufacturing method of the oxygenator 100 will be described.

First, the core 20 is set in a coupling member connected to a motor of a manufacturing apparatus, and the hollow fiber membrane 31 is spirally wound around the outer periphery of the core 20 while the core 20 is rotated. The number of hollow fiber membranes 31 discharged by the manufacturing apparatus may be one or plural (about two to six), and when the number is one, a winding interval of the hollow fiber membranes 31 when winding can be optionally adjusted.

When the winding of the hollow fiber membrane 31 is finished, the outer cylinder 11 is attached, both ends of the hollow fiber membrane layer 30 are fixed with urethane, and then both ends are cut. By this process, the end of the hollow fiber membrane layer 30 is exposed, so that oxygen can enter.

Then, the oxygenator 100 is obtained by attaching the distal end and the proximal end of the outer cylinder 11, the lid 13 and the like.

### [Second Embodiment]

Next, an oxygenator according to a second embodiment of the present invention will be described. Note that, in the following description of the embodiment, differences from the above-described embodiment will be mainly described, and components having functions equivalent to those of the first embodiment will be denoted by the same or related reference numerals and will not be described in detail. The configuration, the members, the method of use and the like may be similar to those in the first embodiment.

An oxygenator 100A of the second embodiment has a configuration in which two layers of hollow fiber membrane layers 30 are provided, gas exchange is performed in one layer, and heat exchange is performed in the other layer. Therefore, the hollow fiber membrane layer 30 of the oxygenator 100A includes a gas exchange unit 32 and a heat exchange unit 33.

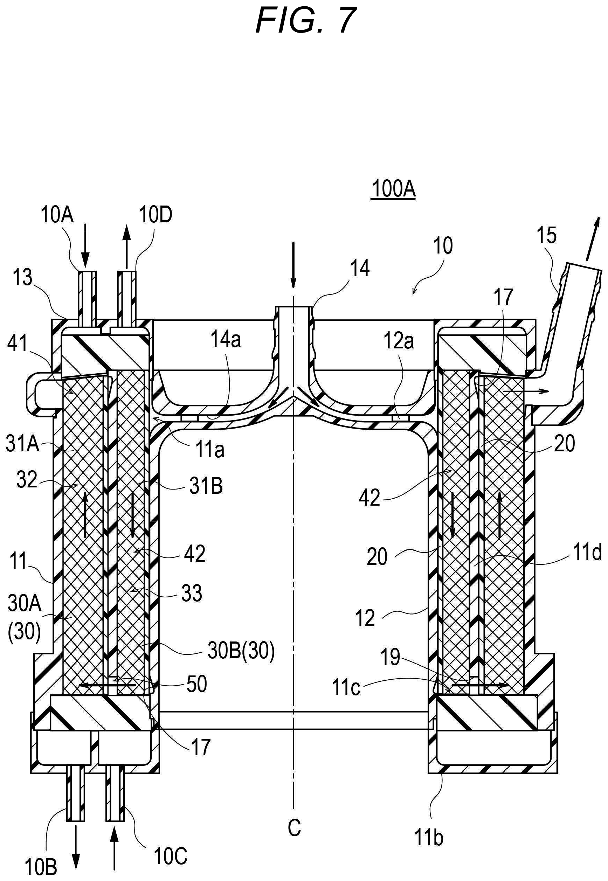

As illustrated in Fig. 7, the oxygenator 100A includes a housing 10, a core 20, the hollow fiber membrane layer 30, a first blood chamber 41, a second blood chamber 42, and a blood flow path 50. A blood inflow port 14 communicates with the second blood chamber 42 via a guide portion 14a. A blood outflow port 15 communicates with the first blood chamber 41 and is formed on a distal end side of an outer cylinder 11 on a downstream side in a blood circulation direction of the blood flow path 50.

The outer cylinder 11 of the housing 10 includes a partition wall 11d that separates a hollow fiber membrane layer 30 (first hollow fiber membrane layer 30A) for gas exchange and a hollow fiber membrane layer 30 (second hollow fiber membrane layer 30B) for heat exchange from each other in an accommodation space 11a that accommodates both of them.

The partition wall 11d is formed over an entire circumference of the outer cylinder 11 in a circumferential direction, and separates the accommodation space 11a into two spaces. Similarly to the inner cylinder 12, the partition wall 11d functions as an annular inner peripheral portion of the housing 10.

On a downstream side in the blood circulation direction of the partition wall 11d, a communication hole 19 that communicates the two spaces separated by the partition wall 11d is formed. The communication hole 19 allows the blood circulating in the second blood chamber 42 to circulate in the first blood chamber 41. The communication hole 19 is an outflow portion of the blood in the blood flow path 50 in the housing 10.

In addition to a gas inflow port 10A and a gas outflow port 10B, the housing 10 of the oxygenator 100A includes a heat medium inflow port 10C that allows a heat medium to flow into the heat exchange unit 33 and a heat medium outflow port 10D that allows the heat medium subjected to the heat exchange to flow out from the heat exchange unit 33. As a result, in the oxygenator 100A, the blood flowing in from the blood inflow port 14 is subjected to the gas exchange when passing through a hollow fiber membrane 31A of the first hollow fiber membrane layer 30A when circulating in the gas exchange unit 32, and is subjected to the heat exchange when passing through a hollow fiber membrane 31B of the second hollow fiber membrane layer 30B when circulating in the heat exchange unit 33. Note that, as illustrated in Fig. 7, the second hollow fiber membrane layer 30B accommodated in the heat exchange unit 33 may be disposed so as to be provided in an entire second blood chamber 42 corresponding to the heat exchange unit 33, or may be disposed so as to form a partial space. That is, the second hollow fiber membrane layer 30B can be appropriately changed according to specifications of the heat exchange unit 33.

From the viewpoint of improving efficiency of the gas exchange in the blood, the gas inflow port 10A is preferably disposed at a position (distal end side of the outer cylinder 11 on a downstream side in the circulation direction of the blood) at which the gas can flow into the first blood chamber 41 opposed to the circulation direction of the blood circulating in the housing 10. The gas outflow port 10B is preferably disposed at a position substantially opposite to the gas inflow port 10A in the longitudinal direction (on the proximal end side of the outer cylinder 11 on an upstream side in the circulation direction of the blood). Note that, the arranging positions of the gas inflow port 10A and the gas outflow port 10B are not limited to the positions illustrated in Fig. 7 as long as they are positions at which heat exchange of blood is possible in the first blood chamber 41.

From the viewpoint of improving efficiency of the heat exchange in the blood, the heat medium inflow port 10C is preferably disposed at a position (proximal end side of the outer cylinder 11 on a downstream side in the circulation direction of the blood) at which the heat medium can flow into the second blood chamber 42 opposed to the circulation direction of the blood circulating in the housing 10. The heat medium outflow port 10D is preferably disposed at a position substantially opposite to the heat medium inflow port 10C in the longitudinal direction (on the distal end side of the outer cylinder 11 on an upstream side in the circulation direction of the blood). Note that, the arranging positions of the heat medium inflow port 10C and the heat medium outflow port 10D are not limited to the positions illustrated in Fig. 7 as long as they are positions at which heat exchange of blood is possible in the second blood chamber 42.

In the oxygenator 100A of the second embodiment, the raised portions 16 include an outer raised portion 16a and an inner raised portion 16b.

The outer raised portion 16a is formed on an inner peripheral surface of the outer cylinder 11 and an inner peripheral surface of the partition wall 11d of the outer cylinder 11. That is, one outer raised portion 16a is formed at a position facing an outermost layer of the first hollow fiber membrane layer 30A accommodated in the first blood chamber 41 functioning as the gas exchange unit 32, and one outer raised portion 16a is formed at a position facing an outermost layer of the second hollow fiber membrane layer 30B accommodated in the second blood chamber 42 functioning as the heat exchange unit 33.

The inner raised portion 16b is formed on an outer peripheral surface of the inner cylinder 12 and an outer peripheral surface of the partition wall 11d of the outer cylinder 11. That is, in the oxygenator 100A of the second embodiment, as illustrated in Fig. 7, one inner raised portion 16b is formed at a position facing an inner peripheral surface of the core 20 in an innermost layer of the first hollow fiber membrane layer 30A accommodated in the first blood chamber 41 functioning as the gas exchange unit 32, and one inner raised portion 16b is formed at a position facing an inner peripheral surface of the core 20 in an innermost layer of the second hollow fiber membrane layer 30B accommodated in the second blood chamber 42 functioning as the heat exchange unit 33.

Note that, it is sufficient that the inner raised portion 16b is formed at least at a position facing the surface of the innermost layer of the first hollow fiber membrane layer 30A accommodated in the first blood chamber 41, that is, on the outer peripheral surface of the partition wall 11d in the first blood chamber 41.

The recess 17 is disposed adjacent to the downstream end of the inner raised portion 16b in the blood circulation direction. In the second embodiment, as illustrated in Fig. 7, the recess 17 is formed on the proximal end side of the inner raised portion 16b formed on the outer peripheral surface of the inner cylinder 12 at a position facing the communication hole 19. The recess 17 is formed at a position on the distal end side of the inner raised portion 16b formed on the outer peripheral surface of the partition wall 11d and facing the proximal end opening of the blood outflow port 15. Each of the communication hole 19 and the proximal end opening of the blood outflow port 15 is an outflow portion of the blood in the blood flow path 50 in the housing 10 in which the circulation direction of the blood rapidly changes from the longitudinal direction to a direction intersecting the longitudinal direction. Therefore, in the blood flowing from the second blood chamber 42 to the first blood chamber 41 and the blood flowing from the first blood chamber 41 to the blood outflow port 15, formation of a stagnation portion due to a rapid change in the circulation direction is suppressed by the recess 17.

Next, the flow of the blood in the oxygenator 100A will be described.

In the oxygenator 100A, the blood circulates in the blood flow path 50 from the blood inflow port 14 to the blood outflow port 15 through the guide portion 14a, the second blood chamber 42, and the first blood chamber 41 in this order.

The blood that flows in from the blood inflow port 14 flows through the guide portion 14a and is guided to the second blood chamber 42.

The blood guided to the second blood chamber 42 circulates between the outer peripheral surface of the inner cylinder 12 and the core 20 in the innermost layer of the second hollow fiber membrane layer 30B, between the inner peripheral surface of the partition wall 11d and the outermost layer of the second hollow fiber membrane layer 30B, and in the second hollow fiber membrane layer 30B to expand to flow from the distal end side toward the proximal end side of the housing 10. When passing through the second hollow fiber membrane layer 30B, the blood exchanges heat in the heat exchange unit 33 while moving through the gap of the hollow fiber membranes 31B.

The blood flowing between the inner peripheral surface of the partition wall 11d and the surface of the outermost layer of the second hollow fiber membrane layer 30B in the second blood chamber 42 of the oxygenator 100A passes through the circulation path 18 formed of the outer raised portion 16a to flow from the distal end toward the proximal end of the housing 10 in the outermost layer of the second hollow fiber membrane layer 30B. The blood flowing between the inner peripheral surface of the inner cylinder 12 and the inner peripheral surface of the core 20 of the innermost layer of the second hollow fiber membrane layer 30B passes through the circulation path 18 formed of the inner raised portion 16b to flow from the distal end toward the proximal end of the housing 10 in the innermost layer of the second hollow fiber membrane layer 30B. Therefore, in the oxygenator 100A, the formation of the stagnation portion of the blood in the outermost layer and the innermost layer of the hollow fiber membrane layer 30 is suppressed, and the thrombus is less likely to be formed.

The blood that passes through the second blood chamber 42 flows into the first blood chamber 41 through the communication hole 19.

The blood that flows into the first blood chamber 41 circulates between the inner peripheral surface of the outer cylinder 11 and the outermost layer of the first hollow fiber membrane layer 30A, between the outer peripheral surface of the partition wall 11d and the core 20 of the innermost layer of the first hollow fiber membrane layer 30A, and in the first hollow fiber membrane layer 30A to expand to flow from the proximal end side toward the distal end side of the housing 10. When passing through the first hollow fiber membrane layer 30A, the blood exchanges gas with oxygen in the gas exchange unit 32 while moving through the gap of the hollow fiber membranes 31A.

The blood flowing between the inner peripheral surface of the outer cylinder 11 and the outermost layer of the first hollow fiber membrane layer 30A in the first blood chamber 41 of the oxygenator 100A passes through the circulation path 18 formed of the outer raised portion 16a to flow from the proximal end toward the distal end of the housing 10 in the outermost layer of the hollow fiber membrane layer 30. The blood flowing between the outer peripheral surface of the partition wall 11d and the inner peripheral surface of the core 20 of the innermost layer of the first hollow fiber membrane layer 30A passes through the circulation path 18 formed of the inner raised portion 16b to flow from the proximal end toward the distal end of the housing 10 in the innermost layer of the first hollow fiber membrane layer 30A. Therefore, in the oxygenator 100A, the formation of the stagnation portion of the blood in the outermost layer and the innermost layer of the hollow fiber membrane layer 30 is suppressed, and the thrombus is less likely to be formed.

In this manner, in the oxygenator 100A, the blood flowing in from the blood inflow port 14 flows through the second blood chamber 42 in the longitudinal direction from the distal end side to the proximal end side of the housing 10, flows into the first blood chamber 41 through the communication hole 19, then flows in the longitudinal direction from the proximal end side to the distal end side of the housing 10 opposite to the inflow direction of the blood inflow port 14, and flows out from the blood outflow port 15.

The blood subjected to the gas exchange flows out of the oxygenator 100A from the blood outflow port 15 communicating with the first blood chamber 41 and returns to the human body via the extracorporeal circulation circuit.

### [Operational Effects]

As described above, the oxygenator 100 according to the present embodiment is the oxygenator 100 including the housing 10 having a cylindrical shape and including the blood inflow port 14 and the blood outflow port 15, and the hollow fiber membrane layer 30 formed by winding the hollow fiber membrane and accommodated in the housing 10, the oxygenator in which the blood flowing in from the blood inflow port 14 flows from one end toward the other end in the longitudinal direction of the housing 10 and flows out from the blood outflow port 15, in which the housing 10 includes a plurality of raised portions 16 (inner raised portions 16b) raised radially inward from the outer surface of the annular inner peripheral portion toward the surface of the innermost layer of the hollow fiber membrane layer 30 and extending from the distal end toward the proximal end of the housing 10, and the recess 17 adjacent to the downstream end of the raised portion 16 in the blood circulation direction, and formed continuously in the circumferential direction of the inner peripheral portion so as to face the outflow portion of the blood in the blood flow path 50 communicating with the blood outflow port 15.

The oxygenator 100 includes the raised portion 16 that forms the circulation direction of the blood flowing between the housing 10 and the surface of the innermost layer of the hollow fiber membrane layer 30 from one end toward the other end in the longitudinal direction of the housing 10. The raised portion 16 increases the flow velocity while defining the flow of the blood flowing in the innermost layer of the hollow fiber membrane layer 30 in the housing 10 to flow from one end toward the other end in the longitudinal direction of the housing 10. Therefore, by increasing the flow velocity of the blood flowing through the innermost layer of the hollow fiber membrane layer 30 by the raised portion 16, the oxygenator 100 can suppress the formation of the stagnation portion of the blood in the innermost layer of the hollow fiber membrane layer 30 to enhance the antithrombogenicity. In the blood flowing in the housing 10 in the longitudinal direction, the flow of the blood at the position of the outflow portion (the blood outflow port 15 and the like) in the blood flow path 50 at which the circulation direction of the blood can be rapidly changed by the recess 17 becomes smooth, and it is possible to suppress the formation of the stagnation portion at a place at which the blood easily stagnates.

The raised portion 16 of the oxygenator 100 may be formed to have a chevron shape having a smooth outer peripheral surface in which an amount raised outward from the center decreases in a transverse sectional view orthogonal to the longitudinal direction, and further may be formed to be smoothly connected to the raised portion 16 adjacent in the circumferential direction.

With such a configuration, the oxygenator 100 can prevent stagnation of the blood flowing between the housing 10 and the hollow fiber membrane layer 30, so that antithrombogenicity can be enhanced.

The hollow fiber membrane layer 30 of the oxygenator 100 may include the gas exchange unit 32 that performs gas exchange of the blood and the heat exchange unit 33 that performs heat exchange of the blood, and the raised portion 16 may be arranged to face at least the surface of the innermost layer of the hollow fiber membrane layer 30 forming the gas exchange unit 32.

With such a configuration, the oxygenator 100 includes the raised portion 16 in which at least the circulation direction of the blood flowing between the surface of the innermost layer of the hollow fiber membrane layer 30 forming the gas exchange unit 32 and the outer peripheral surface of the partition wall 11d to be the inner peripheral portion of the housing 10 is directed from the proximal end side toward the distal end ide of the housing 10. The raised portion 16 increases the flow velocity while defining the flow of the blood flowing in the innermost layer of the hollow fiber membrane layer 30 in the housing 10 to flow from one end toward the other end in the longitudinal direction of the housing 10. Therefore, by increasing the flow velocity of the blood flowing through the innermost layer of the hollow fiber membrane layer 30 forming the gas exchange unit 32 by the raised portion 16, the oxygenator 100 can suppress the formation of the stagnation portion of the blood in the innermost layer of the hollow fiber membrane layer 30 to enhance the antithrombogenicity.

The recess 17 of the oxygenator 100 can include the straight portion 17a of which outer diameter gradually decreases from the downstream end in the blood circulation direction of the raised portion 16, and the curved portion 17b disposed adjacent to the straight portion 17a and having an arc shape directed radially outward with a predetermined curvature radius.

With such a configuration, after the blood flowing through the blood flow path 50 flows along the straight portion 17a, a smooth flow is formed toward the outflow portion of the blood (the proximal end opening of the blood outflow port 15, the communication hole 19 and the like) by the curved portion 17b, so that the formation of the stagnation portion can be effectively suppressed.

The raised portions 16 of the oxygenator 100 may be formed continuously or discontinuously from the distal end to the proximal end of the housing 10.

Even when the raised portions 16 are formed continuously or discontinuously from the distal end to the proximal end of the housing 10, the oxygenator 100 can cause the blood flowing in the housing 10 to have a flow from one end to the other end in the longitudinal direction of the housing 10, so that formation of a stagnation portion in the housing 10 is suppressed, and antithrombogenicity can be improved.

The present application is based on Japanese Patent Application No. 2023-164701 filed on September 27, 2023, the disclosed content of which is incorporated herein by reference in its entirety.

### Reference Signs List

10 Housing
10A Gas inflow port
10B Gas outflow port
10C Heat medium inflow port
10D Heat medium outflow port
11 Outer cylinder (11a accommodation space, 11b outer bottom, 11c inner bottom, 11d partition wall)
12 Inner cylinder (12a protrusion)
13 Lid
14 Blood inflow port
15 Blood outflow port
16 Raised portion (16a apex)
17 Recess
18 Circulation path
19 Communication hole
20 Core
30 Hollow fiber membrane layer
30A First hollow fiber membrane layer
30B Second hollow fiber membrane layer
31, 31A, 31B Hollow fiber membrane
32 Gas exchange unit
33 Heat exchange unit
40 Blood chamber
41 First blood chamber
42 Second blood chamber
50 Blood flow path
100, 100A Oxygenator
C Central axis

## Claims

1. An oxygenator comprising:
a housing having a cylindrical shape and including a blood inflow port and a blood outflow port; and
a hollow fiber membrane layer formed by winding a hollow fiber membrane and accommodated in the housing,
the oxygenator in which blood flowing in from the blood inflow port flows from one end toward the other end in a longitudinal direction of the housing and flows out from the blood outflow port,
wherein the housing includes:
a plurality of raised portions that is raised radially inward from an outer surface of an annular inner peripheral portion toward a surface of an innermost layer of the hollow fiber membrane layer and extends from a distal end toward a proximal end of the housing; and
a recess adjacent to a downstream end of the raised portions in a blood circulation direction and continuously formed in a circumferential direction of the inner peripheral portion so as to face an outflow portion of the blood in a blood flow path communicating with the blood outflow port.

2. The oxygenator according to claim 1, wherein a raised portion has a chevron shape having a smooth outer peripheral surface in which an amount raised outward from a center decreases in a transverse sectional view orthogonal to the longitudinal direction.

3. The oxygenator according to claim 2, wherein the raised portion is smoothly connected with the raised portion adjacent in the circumferential direction.

4. The oxygenator according to claim 1, wherein the hollow fiber membrane layer includes a gas exchange unit that performs gas exchange of the blood and a heat exchange unit that performs heat exchange of the blood, and
the raised portions are disposed at least to face the surface of the innermost layer of the hollow fiber membrane layer forming the gas exchange unit.

5. The oxygenator according to claim 1, wherein the recess includes: a straight portion an outer diameter of which gradually decreases from the downstream end of the raised portions in a blood circulation direction; and
a curved portion disposed adjacent to the straight portion and having an arc shape directed radially outward with a predetermined curvature radius.

6. The oxygenator according to any one of claims 1 to 5, wherein the raised portions are formed continuously or discontinuously from the distal end to the proximal end of the housing.
